# EUROPEAN PATENT APPLICATION

(11) **EP 1 897 547 A1**
(43) Date of publication of application: **12.03.2008**
(21) Application number: 07011057.2
(22) Date of filing: 13.07.2000
(51) Int. Cl.: A61K 31/675, A61P 3/10

(54) **Use of pyridoxal phosphate derivatives for the treatment of diabetes and related complications**

(30) Priority: 13.07.1999 US 143466 P
(62) Divisional of application: 04016316.4
(71) Applicant: Medicure Inc., Winnipeg, Manitoba R3T 0P4 (CA)
(72) Inventor: Sethi, Rajat, Winnipeg Manitoba, R2N 3K9 (CA); Haque, Wasimul, Edmonton Alberta, T6L 2Y7 (CA)
(74) Representative: Vossius & Partner

(57) **Abstract**

Use of a 3-acylated pyridoxal analogue, a pharmaceutically acceptable acid addition salt thereof, or a mixture thereof for the preparation of a medicament for treating insulin-dependent or non-insulin-dependent diabetes mellitus, related conditions, symptoms of insulin-dependent or non-insulin-dependent diabetes mellitus or related conditions, or any combination thereof in a mammal.

## Description

### Field of the Invention

This invention relates to methods of treating insulin-dependent diabetes mellitus, noninsulin-dependent diabetes mellitus, and related conditions and symptoms.

### Background

Diabetes mellitus is a condition in which blood glucose levels are abnormally high because the body is unable to produce enough insulin to maintain normal blood glucose levels or is unable to adequately respond to the insulin produced. Insulin-dependent diabetes mellitus (often referred to as type I diabetes) arises when the body produces little or no insulin. About 10% of all diabetics have type I diabetes. Noninsulin-dependent diabetes mellitus (often referred to as type II diabetes) arises when the body cannot adequately respond to the insulin that is produced in response to blood glucose levels. Type II diabetes is often associated with hyperglycemia (high plasma glucose levels due to decreased glucose utilization) and hyperinsulinemia (high plasma insulin levels due to decreased insulin receptors available), factors that contribute to insulin resistance.

Available treatments include weight control, exercise, diet, and drug therapy. Drug therapy, for type I diabetes mellitus requires the administration of insulin; however, drug therapy for type II diabetes mellitus usually involves the administration of insulin and/or oral hypoglycemic drugs to lower blood glucose levels. If the oral hypoglycemic drugs fail to control blood sugar, then insulin, either alone or in combination with the hypoglycemic drugs, will usually be administered.

Although many of the symptoms of diabetes mellitus may be controlled by insulin therapy, the long-term complications of both type I and type II diabetes mellitus are severe and may reduce life expectancy by as much as one third. Over time, elevated blood glucose levels damage blood vessels, eyes, kidneys, nerves, autonomic nervous system, skin, connective tissue, and white blood cell function.

Moreover, insulin therapy may result in insulin allergy, insulin resistance, atrophy of the subcutaneous fat at the site of insulin injection (i.e., lipoatrophy), enlargement of subcutaneous fat deposit (i.e., lipohypertrophy) due to lipogenic action of high local concentration of insulin, and insulin adema.

Thus, it would be desirable to find an alternative to the above-described therapies or to administer a drug therapy that may reduce the amount of insulin or hypoglycemic drug required, yet maintain the effectiveness of the insulin or hypoglycemic drug administered.

### Summary of the Invention

The present invention provides methods for treating insulin-dependent diabetes mellitus, noninsulin-dependent diabetes mellitus, and related conditions and symptoms. One embodiment includes a method of treating diabetes mellitus in a mammal by administering a therapeutically effective amount of a compound, such as pyridoxal-5'- phosphate, pyridoxal, pyridoxamine, pyridoxine, a 3-acylated pyridoxal analogue, a pharmaceutically acceptable acid addition salt thereof, or a mixture thereof.

In another embodiment, the invention provides a method of treating diabetes mellitus in a mammal by concurrently administering a therapeutically effective amount of a combination of insulin and a compound, such as pyridoxal-5'-phosphate, pyridoxal, pyridoxamine, pyridoxine, a 3-acylated pyridoxal analogue, a pharmaceutically acceptable acid addition salt thereof, or a mixture thereof.

In still another embodiment, the invention provides a method of treating noninsulin-dependent diabetes mellitus in a mammal by concurrently administering a therapeutically effective amount of a combination of a hypoglycemic compound and a compound, such as pyridoxal-5'-phosphate, pyridoxal, pyridoxamine, pyridoxine, a 3-acylated pyridoxal analogue, a pharmaceutically acceptable acid addition salt thereof, or a mixture thereof.

### Brief Description of the Drawings

Figure 1 shows the effect of P-5-P and insulin alone or in combination on plasma glucose levels in rats induced with Type I diabetes.
Figure 2 shows the effect of P-5-P and insulin alone or in combination on plasma insulin levels in rats induced with Type I diabetes.
Figure 3 shows the effect of P-5-P and tolbutamide alone or in combination on plasma glucose levels in rats induced with Type II diabetes.
Figure 4 shows the effect of P-5-P and tolbutamide alone or in combination on plasma insulin levels in rats induced with Type II diabetes.
Figure 5 shows the effect of P-5-P and tolbutamide alone or in combination on increased systolic blood pressure in rats induced with Type II diabetes.

### Detailed Description

The present invention provides methods for treatment of diabetes mellitus and related conditions and symptoms. Such diabetes mellitus and related conditions include insulin-dependent diabetes mellitus (type I diabetes), noninsulin-dependent diabetes mellitus (type II diabetes), insulin resistance, hyperinsulinemia, and diabetes-induced hypertension. Other diabetes-related conditions include obesity and damage to blood vessels, eyes, kidneys, nerves, autonomic nervous system, skin, connective tissue, and immune system.

In accordance with the present invention, it has been found that compounds, such as, for example, pyridoxal-5'-phosphate, pyridoxal, pyridoxamine, a 3-acylated pyridoxal analogue, a pharmaceutically acceptable acid addition salt thereof, or a mixture thereof, either alone or in combination with insulin and/or hypoglycemic compounds can be used in the treatment of the above-identified diseases and conditions. As used herein, "treatment" and "treating" include preventing, inhibiting, and alleviating diabetes mellitus and related conditions and symptoms. In some instances, the treatment may be carried out by administering a therapeutically effective amount of a compound suitable for use in methods of the invention. In other instances, the treatment may be carried out by concurrently administering a therapeutically effective amount of a combination of insulin and a compound suitable for use in methods of the invention. In still other instances, the treatment may involve concurrently administering a therapeutically effective amount of a combination of a hypoglycemic compound and a compound suitable for use in methods of the invention when the diabetes mellitus and related conditions to be treated is type II diabetes, insulin resistance, hyperinsulinemia, diabetes-induced hypertension, obesity, or damage to blood vessels, eyes, kidneys, nerves, autonomic nervous system, skin, connective tissue, or immune system.

A "therapeutically effective amount" as used herein includes a prophylactic amount, for example, an amount effective for preventing or protecting against diabetes mellitus and related conditions and symptoms, and amounts effective for alleviating or healing diabetes mellitus and related conditions and symptoms Generally, by administering a compound suitable for use in methods of the invention concurrently with insulin and/or a hypoglycemic compound the insulin and/or hypoglycemic compound may be administered in a dosage amount that is less than the dosage amount required when the insulin and/or hypoglycemic compound is the sole active ingredient By administering lower dosage amounts of insulin and/or a hypoglycemic compound, the side effects associated therewith should accordingly be reduced and/or the onset of the long-term complications that arise from diabetes mellitus and related conditions may be delayed.

Compounds suitable for use in the methods of the invention include pyridoxal-5'-phosphate, pyridoxal, pyridoxamine, pyridoxine, 3-acylated pyridoxal analogues, pharmaceutically acceptable acid addition salts thereof, or a mixture thereof. 3-Acylated pyridoxal analogues include prodrugs of pyridoxal that provide for slower metabolism to pyridoxal *in vivo.* For example, one suitable 3-acylated analogue of pyridoxal (2-methyl-3-hydroxy-4-formyl-5-hydroxymethylpyridine) is a compound of the formula I: 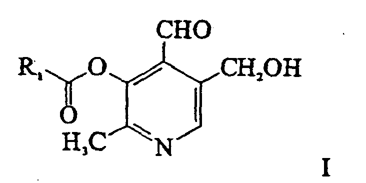 or a pharmaceutically acceptable acid addition salt thereof, wherein

R₁ is a straight or branched alkyl group, a straight or branched alkenyl group, in which an alkyl or alkenyl group may be interrupted by a nitrogen or oxygen atom; an alkoxy group; a dialkylamino group; or an unsubstituted or substituted aryl group.

The term "alkyl" group includes a straight or branched saturated aliphatic hydrocarbon chain having from 1 to 8 carbon atoms, such as, for example, methyl, ethyl, propyl, isopropyl (1-methylethyl), butyl, *tert-*butyl (1,1-dimethylethyl), and the like.

The term "alkenyl" group includes an unsaturated aliphatic hydrocarbon chain having from 2 to 8 carbon atoms, such as, for example, ethenyl, 1-propenyl, 2-propenyl, 1-butenyl, 2-methyl-1-propenyl, and the like.

The above alkyl or alkenyl groups may optionally be interrupted in the chain by a heteroatom, such as, for example, a nitrogen or oxygen atom, forming an alkylaminoalkyl or alkoxyalkyl group, for example, methylaminoethyl or methoxymethyl, and the like.

The term "alkoxy" group includes an alkyl group as defined above joined to an oxygen atom having preferably from 1 to 4 carbon atoms in a straight or branched chain, such as, for example, methoxy, ethoxy, propoxy, isopropoxy (1-methylethoxy), butoxy, tert-butoxy (1,1-dimethylethoxy), and the like.

The term "dialkylamino" group includes two alkyl groups as defined above joined to a nitrogen atom, in which the alkyl group has preferably 1 to 4 carbon atoms, such as, for example, dimethylamino, diethylamino, methylethylamino, methylpropylamino, diethylamino, and the like.

The term "aryl" group includes an aromatic hydrocarbon group, including fused aromatic rings, such as, for example, phenyl and naphthyl. Such groups may be unsubstituted or substituted on the aromatic ring by, for example, an alkyl group of 1 to 4 carbon atoms, an alkoxy group of 1 to 4 carbon atoms, an amino group, a hydroxy group, or an acetyloxy group.

Preferred R₁ groups for compounds of formula I are toluyl or naphthyl. Such R₁ groups when joined with a carbonyl group form an acyl group 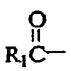 which preferred for compounds of formula I include toluoyl or β-naphthoyl. Of the toluoyl group, the p-isomer is more preferred.

Examples of 3-acylated analogues of pyridoxal include, but are not limited to, 2-methyl-3-toluoyloxy-4-formyl-5-hydroxymethylpyridine and 2-methyl-β-naphthoyloxy-4-fotmyl-5-hydroxymethylpyridine.

Another suitable analogue is a 3-acylated analogue of pyridoxal-4,5-aminal (1-secondary amino-1,3-dihydro-7-hydroxy-6-methylfuro(3,4-c)pyridine) of the formula II: 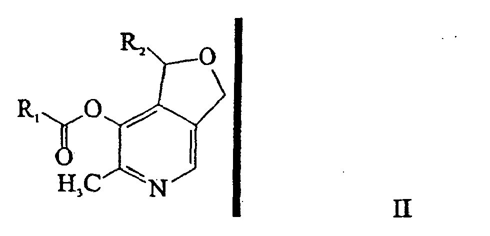 or a pharmaceutically acceptable acid addition salt thereof, wherein

R₁ is a straight or branched alkyl group, a straight or branched alkenyl group, in which an alkyl or alkenyl group may be interrupted by a nitrogen or oxygen atom; an alkoxy group; a dialkylamino group; or an unsubstituted or substituted aryl group; and

R₂ is a secondary amino group.

The terms "alkyl," "alkenyl, " "alkoxy," "dialkylamino," and "aryl" are as defined above.

The term "secondary amino" group includes a group of the formula III: 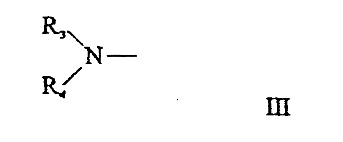 derived from a secondary amine R₃R₄NH, in which R₃ and R₄ are each independently alkyl, alkenyl, cycloalkyl, aryl, or, when R₃ and R₄ are taken together, may form a ring with the nitrogen atom and which may optionally be interrupted by a heteroatom, such as, for example, a nitrogen or oxygen atom. The terms "alkyl," "alkenyl," and "aryl" are used as defined above in forming secondary amino groups such as, for example, dimethylamino, methylethylamino, diethylamino, dialkylamino, phenylmethylamino, diphenylamino, and the like.

The term "cycloalkyl" refers to a saturated hydrocarbon having from 3 to 8 carbon atoms, preferably 3 to 6 carbon atoms, such as, for example, cyclopropyl, cyclopentyl, cyclohexyl, and the like.

When R₃ and R₄ are taken together with the nitrogen atom, they may form a cyclic secondary amino group, such as, for example, piperidino, and, when interrupted with a heteroatom, includes, for example, piperazino and morpholino.

Preferred R₁ groups for compounds of formula II include toluyl, e.g., *p-*toluyl, naphthyl, ten-butyl, dimethylamino, acetylphenyl, hydroxyphenyl, or alkoxy, e.g., methoxy. Such R₁ groups when joined with a carbonyl group form an acyl group 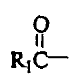 which preferred for compounds and formula II include toluoyl, β-naphthoyl, pivaloyl, dimethylcarbamoyl, acetylsalicyloyl, salicyloyl, or alkoxycarbonyl. A preferred secondary amino group may be morpholino.

Examples of 3-acylated analogues of pyridoxal-4,5-aminal include, but are not limited to, 1-morpholino-1,3-dihydro-7-(*p*-toluoyloxy)-6-methylfuro(3,4-*c*)pyridine; 1-morpholino-1,3-dihydro-7-(β-naphthoyloxy)-6-methylfuro(3,4-*c*)pyridine; 1 -morpholino-1,3-dihydro-7-pivaloyloxy-6-methylfuro(3,4-*c*)pyridine; 1-morpholino-1,3-dihydro-7-carbamoyloxy-6-methylfuro(3,4-*c*)pyridine; and 1-morpholino-1,3-dihydro-7-acetylsalicyloxy-6-methylfuro(3,4-*c*)pyridine.

The compounds of formula I may be prepared by reacting pyridoxal hydrochloride with an acyl halide in an aprotic solvent. A suitable acyl group is 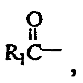 wherein R₁ is as defined above. A particularly suitable acyl halide includes p-toluoyl chloride or β-naphthoyl chloride. A suitable aprotic solvent includes acetone, methylethylketone, and the like.

The compounds of formula II may be prepared by reacting 1-secondary amino-1,3-dihydro-7-hydroxy-6-methylfuro(3,4-*c*)pyridine with an acyl halide in an aprotic solvent. An acyl group is 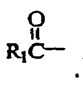 wherein R₁ is as defined above. A particularly suitable acyl halide includes p-toluoyl chloride, β-naphthoyl chloride, trimethylacetyl chloride, dimethylcarbamoyl chloride, and acetylsalicyloyl chloride. A particularly suitable secondary amino group includes morpholino.

The compound 1-morpholino-1,3-dihydro-7-hydroxy-6-methylfuro(3,4-*c*)pyridine may be prepared by methods known in the art, for example, by reacting morpholine and pyridoxal hydrochloride at a temperature of about 100°C in a solvent. A suitable solvent includes, for example, toluene. Similarly, other secondary amines as defined for R₂ may be used as reactants to prepare the appropriate 1-secondary amino compounds.

The compounds of formula I may alternatively be prepared from the compounds of formula II by reacting a compound of formula II with an aqueous acid, such as, for example, aqueous acetic acid.

One skilled in the art would recognize variations in the sequence and would recognize variations in the appropriate reaction conditions from the analogous reactions shown or otherwise known that may be appropriately used in the above-described processes to make the compounds of formulas I and II herein.

The products of the reactions described herein are isolated by conventional means such as extraction, distillation, chromatography, and the like.

Pharmaceutically acceptable acid addition salts of the compounds suitable for use in methods of the invention include salts derived from nontoxic inorganic acids such as hydrochloric, nitric, phosphoric, sulfuric, hydrobromic, hydriodic, hydrofluoric, phosphorous, and the like, as well as the salts derived from nontoxic organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate, n-methyl glutamine, etc. (see, e.g., Berge et al., J. Pharmaceutical Science, 66: 1-19 (1977).

The acid addition salts of the basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

A physician or veterinarian of ordinary skill readily determines a subject who is exhibiting symptoms of any one or more of the diseases described above. Regardless of the route of administration selected, the compounds suitable for use in methods of the invention are formulated into pharmaceutically acceptable unit dosage forms by conventional methods known to the pharmaceutical art. An effective but nontoxic quantity of the compound is employed in treatment. The compounds can be administered in enteral unit dosage forms, such as, for example, tablets, sustained release tablets, enteric coated tablets, capsules, sustained release capsules, enteric coated capsules, pills, powders, granules, solutions, and the like. They may also be administered parenterally, such as, for example, subcutaneously, intramuscularly, intradermally, intramammarally, intravenously, and other administrative methods known in the art.

Although it is possible for a compound suitable for use in methods of the invention to be administered alone in a unit dosage form, preferably the compound is administered in admixture as a pharmaceutical composition suitable for use in methods of the invention. A pharmaceutical composition comprises a pharmaceutically acceptable carrier and a compound. A pharmaceutically acceptable carrier includes, but is not limited to, physiological saline, ringers, phosphate buffered saline, and other carriers known in the art. Pharmaceutical compositions may also include additives, for example, stabilizers, antioxidants, colorants, excipients, binders, thickeners, dispersing agents, readsorpotion enhancers, buffers, surfactants, preservatives, emulsifiers, isotonizing agents, and diluents. Pharmaceutically acceptable carriers and additives are chosen such that side effects from the pharmaceutical compound are minimized and the performance of the compound is not canceled or inhibited to such an extent that treatment is ineffective.

Methods of preparing pharmaceutical compositions containing a pharmaceutically acceptable carrier and a compound suitable for use in methods of the invention are known to those of skill in the art. All methods may include the step of bringing the compound in association with the carrier and additives. In general, the formulations are prepared by uniformly and intimately bringing the compound of the invention into association with a liquid carrier or a finely divided solid carrier or both, and then, if necessary, shaping the product into the desired unit dosage form.

The ordinarily skilled physician or veterinarian will readily determine and prescribe the therapeutically effective amount of the compound to treat the disease for which treatment is administered. In so proceeding, the physician or veterinarian could employ relatively low dosages at first, subsequently increasing the dose until a maximum response is obtained. Typically, the particular disease, the severity of the disease, the compound to be administered, the route of administration, and the characteristics of the mammal to be treated, for example, age, sex, and weight, are considered in determining the effective amount to administer. Generally, a therapeutically effective amount of a compound to treat diabetes mellitus and related conditions and symptoms is in a range of about 0.1-100 mg/kg of a patient's body weight, more preferably in the range of about 0.5-50 mg/kg of a patient's body weight, per daily dose. The compound may be administered for periods of short and long duration.

A therapeutically effective amount of a compound for treating diabetes mellitus and related conditions and symptoms can be administered prior to, concurrently with, or after the onset of the disease or symptom. The compound can be administered to treat diabetes mellitus type I, diabetes mellitus type II, or obesity. Preferably, the compound can be administered to treat damage to blood vessels, eyes, kidneys, nerves, autonomic nervous system, skin, connective tissue, or immune system. Still preferably, the compound can be administered to treat insulin resistance or hyperinsulinemia. Also preferably, the compound can be administered to treat diabetes-induced hypertension.

Moreover, the compound may be administered concurrently with insulin and/or a hypoglycemic compound to treat diabetes mellitus and related conditions and symptoms. The compound can be administered concurrently with insulin and/or a hypoglycemic compound to treat type I diabetes, type II diabetes, or obesity. Preferably, the compound can be administered concurrently with insulin and/or hypoglycemic compound to treat damage to blood vessels, eyes, kidneys, nerves, autonomic nervous system, skin, connective tissue, or immune system. Still preferably, the compound can be administered concurrently with insulin and/or hypoglycemic compound to treat insulin resistance or hyperinsulinemia. Also preferably, the compound can be administered concurrently with insulin and/or hypoglycemic compound to treat diabetes-induced hypertension.

Typically, a compound may be administered concurrently with insulin to treat type I diabetes, type II diabetes, and related conditions and symptoms. For type II diabetes, insulin resistance, hyperinsulinemia, diabetes-induced hypertension, obesity, or damage to blood vessels, eyes, kidneys, nerves, autonomic nervous system, skin, connective tissue, or immune system, a compound may be administered concurrently with a hypoglycemic compound instead of insulin. Alternatively, a compound may be administered concurrently with insulin and a hypoglycemic compound to treat type II diabetes, insulin resistance, hyperinsulinemia, diabetes-induced hypertension, obesity, or damage to blood vessels, eyes, kidneys, nerves, autonomic nervous system, skin, connective tissue, or immune system.

"Concurrent administration" and "concurrently administering" as used herein includes administering a compound suitable for use in methods of the invention and insulin and/or a hypoglycemic compound in admixture, such as, for example, in a pharmaceutical composition, or as separate compounds, such as, for example, separate pharmaceutical compositions administered consecutively, simultaneously, or at different times. Preferably, if the compound and insulin and/or hypoglycemic compound are administered separately, they are not administered so distant in time from each other that the compound and the insulin and/or hypoglycemic compound cannot interact and a lower dosage amount of insulin and/or hypoglycemic compound cannot be administered.

Suitable hypoglycemic compounds include, for example, metformin, acarbose, acetohexamide, glimepiride, tolazamide, glipizide, glyburide, tolbutamide, chlorpropamide, thiazolidinediones, alpha glucosidase inhibitors, biguanindine derivatives, and troglitazone, and a mixture thereof. Preferably, the hypoglycemic compound is tolbutamide.

A physician or veterinarian of ordinary skill readily determines a subject who is exhibiting symptoms of diabetes mellitus and related conditions and symptoms. Regardless of the route of administration selected, the compound and the insulin and/or hypoglycemic compound are formulated into pharmaceutically acceptable unit dosage forms by conventional methods known to the pharmaceutical art. An effective but nontoxic quantity of the compound and the insulin and/or hypoglycemic compound is employed in the treatment of diabetes mellitus and related conditions and symptoms. The compound and the insulin may be concurrently administered parenterally in admixture or may be concurrently administered enterally and/or parenterally separately. Similarly, the compound and the hypoglycemic compound may be concurrently administered enterally in admixture or may be administered enterally and/or parenterally separately. In some instances, the compound may be concurrently administered with insulin and a hypoglycemic compound. Such administration would involve enteral and/or parenteral administration as described above.

Parenteral administration includes subcutaneous, intramuscular, intradermal, intramammary, intravenous, and other administrative methods known in the art. Enteral administration includes tablets, sustained release tablets, enteric coated tablets, capsules, sustained release capsules, enteric coated capsules, pills, powders, granules, solutions, and the like.

A pharmaceutical composition suitable for administration includes a pharmaceutically acceptable carrier and a compound suitable for use in methods of the invention and, optionally, insulin and/or a hypoglycemic compound. A pharmaceutically acceptable carrier includes, but is not limited to, physiological saline, ringers, phosphate buffered saline, and other carriers known in the art. Pharmaceutical compositions may also include stabilizers, antioxidants, colorants, and diluents. Pharmaceutically acceptable carriers and additives are chosen such that side effects from the pharmaceutical compound are minimized and the performance of the compound is not canceled or inhibited to such an extent that treatment is ineffective.

Methods of preparing pharmaceutical compositions containing a pharmaceutically acceptable carrier and a compound suitable for use in methods of the invention and, optionally, insulin and/or a hypoglycemic compound, are known to those of skill in the art. All methods may include the step of bringing the compound and, optionally, a hypoglycemic compound in association with the carrier or additives. In general, the formulations are prepared by uniformly and intimately bringing the compound into association with a liquid carrier or a finely divided solid carrier or both, and then, if necessary, shaping the product into the desired unit dosage form.

The ordinarily skilled physician or veterinarian will readily determine and prescribe the therapeutically effective amount of the compound to treat the disease for which treatment is administered as described above.

When concurrently administering a compound with insulin and/or a hypoglycemic compound, the compound is administered in a range of about 0.1-100 mg per daily dose, typically 0.5-50 mg/kg of body weight per daily dose. A hypoglycemic compound is administered in a range of about 1 to 300 mg per daily dose, typically 1 to 200 mg per daily dose. Insulin is typically administered in a range of about 0.1 to 2 units/kg of a patient's body weight per daily dose. A "unit" of insulin refers to that amount of insulin necessary to lower the blood-sugar level in a rabbit by 50% in I to 3 hours.

The invention is further elaborated by the representative examples as follows. Such examples are not meant to be limiting but only illustrative of the invention.

### EXAMPLES

### Example 1: Synthesis of morpholine pyridoxal-4,5-aminal (1-morpholino-1,3-dihydro-7-hydroxy-6-methylfuro(3,4-c)pyridine)

A mixture of morpholine (20g) and toluene (100mL) was stirred and heated using an oil bath set to 100°C for 15 minutes. Pyridoxal hydrochloride (10g) was then added and the reaction mixture was stirred at 100°C for two hours. The reaction mixture was then concentrated by distillation of the toluene and morpholine. The concentrated reaction mixture was washed three times by adding toluene (100mL) and removing the toluene by distillation. After washing, the residue was dissolved in toluene and filtered, and then hexane was added until precipitation began, at which time the reaction mixture was left overnight at room temperature. Crystals were collected and washed thoroughly with hexane.

Nuclear magnetic resonance spectroscopy (NMR) and mass spectroscopy confirmed the identity of the synthesized compound. The purity of the compound was analyzed by high performance liquid chromatography (HPLC) using a C-18 reverse phase column and water/acetonitrile as solvent (1-100% acetonitrile over 25 minutes).

### Example 2: Synthesis of the 3-toluate of the morpholine pyridoxal-4,5-aminal (1-morpholino-1,3-dihydro-7-(p-toluoyloxy)-6-methylfuro(3,4-c)pyridine)

Anhydrous powdered potassium carbonate (5g), acetone (100mL), and morpholine pyridoxal-4,5-aminal (1-morpholino-1,3-dihydro-7-hydroxy-6-methylfuro(3,4-*c*)pyridine) (1.11 g, 5 mmoles) were mixed in a nitrogen-cooled, dry flask. The reaction mixture was cooled to between 0 and 5°C and then p-toluoyl chloride (1.06g, 6 mmoles) in acetone (20mL) was added. This mixture was stirred for two hours, followed by filtering out the solid and evaporating the solution to dryness under vacuum. The residue was chromatographed on silica gel using a mixture of ethyl acetate and hexane as solvent.

The purified solid was analyzed by thin layer chromatography (TLC), NMR, and mass spectroscopy. The purity of the synthesized compound was confirmed by HPLC as described in Example 1.

### Example 3: Synthesis of the 3-toluate of pyridoxal (2-methyl-3-toluoyloxy-4-formyl-5-hydroxymethylpyridine)

Anhydrous potassium carbonate (10g), acetone (100mL), and pyridoxal hydrochloride (2.03g, 10 mmoles) were mixed in a nitrogen-cooled, dry flask. The mixture was cooled to between 0 and 5°C and then *p*-toluoyl chloride (2.12g, 12 mmoles) in acetone (20mL) was added. The reaction mixture was stirred for two hours followed by filtering out the solid and evaporating the solution to dryness under vacuum. The residue was chromatographed on silica gel as described in Example 2.

The purified solid was analyzed by TLC, NMR, and mass spectroscopy. The purity of the compound was confirmed by HPLC as described in Example 1.

Alternative to the above-described method, the 3-toluate of pyridoxal is synthesized by reacting the compound of Example 2 with 80% aqueous acetic acid at 60°C for 30 minutes, and then diluting with water and extracting by ethyl acetate. The ethyl acetate layer is washed with 5% aqueous sodium bicarbonate, dried with magnesium sulfate, and evaporated to dryness. The compound is also analyzed as described *supra.*

### Example 4: Synthesis of 3-β-naphthoate of the morpholine pyridoxal-4,5-aminal (1-morpholino-1,3-dihydro-7-(β-naphthoyloxy)-6-methylfuro(3,4-c)pyridine)

Anhydrous powdered potassium carbonate (5g), acetone (100mL), and morpholine pyridoxal-4,5-aminal (1-morpholino-1,3-dihydro-7-hydroxy-6-methylfuro(3,4-c)pyridine) (1.11g, 5 mmoles) were mixed in a nitrogen-cooled, dry flask. The mixture was cooled to between 0 and 5°C and then β-naphthoyl chloride (1.06g, 6 mmoles) in acetone (20mL) was added. The reaction mixture was stirred for two hours, and then the solid was filtered out and the solution was evaporated to dryness under vacuum. The residue was chromatographed according to Example 2.

The purified solid was analyzed according to Example 2, and the purity was confirmed according to Example 1.

### Example 5: Synthesis of the 3-β-naphthoate of pyridoxal (2-methyl-3-β-naphthoyloxy-4-formyl-5-hydroxymethylpyridine)

Anhydrous potassium carbonate (10g), acetone (100mL), and pyridoxal hydrochloride (2.03g, 10 mmoles) were mixed in a nitrogen-cooled, dry flask. The mixture was cooled to between 0 and 5°C and then β-naphthoyl chloride (2.12g, 12 mmoles) in acetone (20mL) was added and the mixture was stirred for two hours. The solid was filtered out and the solution was evaporated to dryness under vacuum. The residue was chromatographed according to Example 2.

The purified solid was analyzed according to Example 2, and the purity was confirmed according to Example 1.

Alternative to the above-described synthesis, the 3-β-naphthoate of pyridoxal is prepared by reacting the compound of Example 4 with 80% aqueous acetic acid at 60°C for 30 minutes, followed by diluting with water and extracting by ethyl acetate. The ethyl acetate layer is then washed with 5% aqueous sodium bicarbonate, dried with magnesium sulfate, and evaporated to dryness. The compound is also analyzed as described *supra.*

### Example 6: Synthesis of 3-pivaloyl of the morpholine pyridoxal-4,5-aminal (1-morpholino-1,3-dihydro-7-pivaloyloxy)-6-methylfuro(3,4-c)pyridine)

Anhydrous powdered potassium carbonate (5g), acetone (100mL), and morpholine pyridoxal-4,5-aminal (1-morpholino-1,3-dihydro-7-hydroxy-6-methylfuro(3,4-c)pyridine) (1.11g, 5 mmoles) were mixed in a nitrogen-cooled, dry flask. The mixture was cooled to between 0 and 5°C and then pivaloyl chloride (trimethylacetyl chloride) (720mg, 6 mmoles) in acetone (20mL) was added. The reaction mixture was stirred for two hours. The solid was then filtered out and the solution was evaporated to dryness under vacuum. The residue was chromatographed according to Example 2.

The purified solid was analyzed according to Example 2, and the purity was confirmed according to Example 1.

### Example 7: Synthesis of 3-dimethylcarbamoyl of the morpholine pyridoxal-4,5-aminal (1-morpholino-1,3-dihydro-7-(dimethylcarbamoyloxy)-6-methylfuro(3,4-c)pyridine)

Anhydrous powdered potassium carbonate (5g), acetone (100mL), and morpholine pyridoxal-4,5-aminal (1-morpholino-1,3-dihydro-7-hydroxy-6-methylfuro(3,4-c)pyridine) (1.11g, 5 mmoles) were mixed in a nitrogen-cooled, dry flask. The mixture was cooled to between 0 and 5°C and then dimethylcarbamoyl chloride (642mg, 6 mmoles) in acetone (20mL) was added. The reaction mixture was stirred for two hours. The solid was then filtered out and the solution was evaporated to dryness under vacuum. The residue was chromatographed according to Example 2.

The purified solid was analyzed according to Example 2, and the purity was confirmed according to Example 1.

### Example 8: Synthesis of 3-acetylsalicyloyl of the morpholine pyridoxal-4,5-aminal (1-morpholino-1,3-dihydro-7-acetylsalicyloxy)-6-methylfuro(3,4-c)pyridine)

Anhydrous powdered potassium carbonate (5g), acetone (100mL), and morpholine pyridoxal-4,5-aminal (1-morpholino-1,3-dihydro-7-hydroxy-6-methylfuro(3,4-c)pyridine) (1.11g, 5 mmoles) were mixed in a nitrogen-cooled, dry flask. The mixture was cooled to between 0 and 5°C and then acetylsalicyloyl chloride (1.09g, 6 mmoles) in acetone (20mL) was added. The reaction mixture was stirred for two hours. The solid was then filtered out and the solution was evaporated to dryness under vacuum. The residue was chromatographed according to Example 2.

The purified solid was analyzed according to Example 2, and the purity was confirmed according to Example 1.

### Example 9: Control of Glucose and Insulin Levels in Subjects Having Type I Diabetes by Administration of Pyridoxal-5'-Phosphate

To determine the effect of a compound suitable for use in methods of the invention on glucose and insulin levels, rats are administered pyridoxal-5'-phosphate, either alone or in combination with insulin, after being experimentally induced with type I diabetes, and their urine and blood glucose and insulin levels are determined.

Male Sprague-Dawley (Charles River Laboratories, Montreal, Canada) rats weighing approximately 200g are randomly separated into control and experimental groups. All experimental animals are given an intravenous injection of 0.1 M citrate buffered streptozotocin (pH 4.5) at a dosage of 65 mg/kg of body weight to induce diabetes mellitus. All control animals receive an intravenous injection of 0.1 M citrate buffer (pH 4.5) alone.

One experimental group of rats also receives daily doses of pyridoxal-5'-phosphate (25 mg/kg body weight) (Aldrich Canada Ltd., Ontario, Canada). A second experimental group receives daily subcutanteous injections of Humulin® N (3 units per day) (insulin isophene, human biosynthetic; Eli Lilly and Co., Indianapolis, Indiana). A third experimental group receives both daily subcutaneous injections of Humulin® N (3 units per day) and a daily dose of pyridoxal-5'-phosphate (25 mg/kg body weight). A fourth experimental group receives daily subcutaneous injections of Humulin® N (1.5 units per day). A fifth experimental group receives both daily subcutaneous injections of Humulin® N (1.5 units per day) and a daily dose of pyridoxal-5'-phosphate (25 mg/kg body weight).

All animals are fed rat chow and water ad libitum. Plasma glucose levels were done using the Infinity Glucose Reagent® (Sigma Diagnostics, St. Louis, MO).

The experimental group of rats that receive daily doses of pyridoxal-5'-phosphate and daily injections of Humulin® N show reduced levels of glucose and insulin in blood and urine samples when compared with the group of rats that receive daily injections of Humulin® N without receiving daily doses of pyridoxal-5'-phosphate.

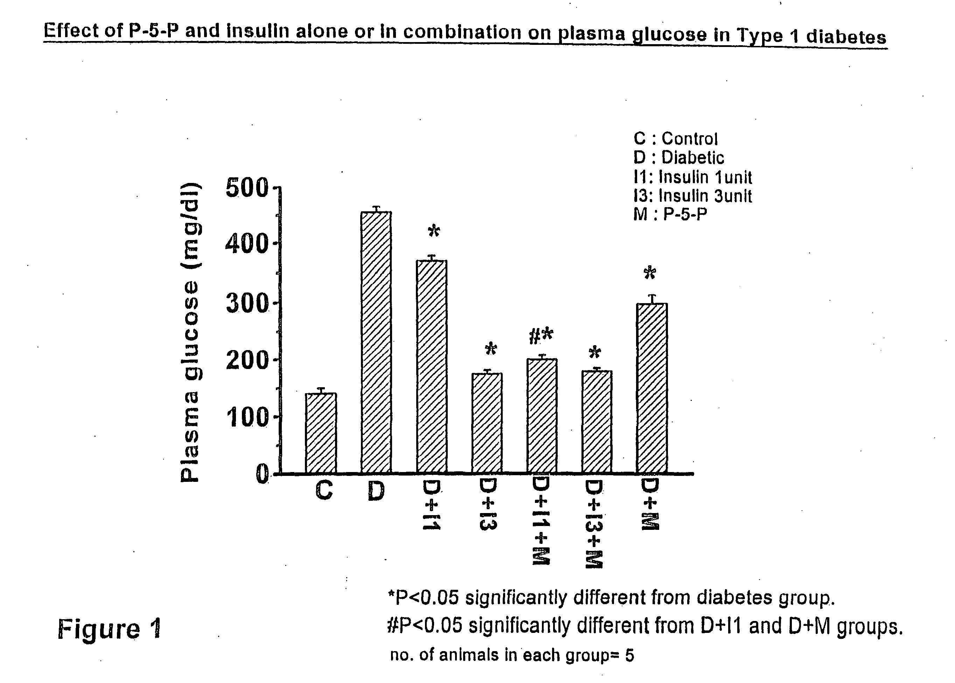

Figure 1 demonstrates the effect of P-5-P and insulin alone or in combination on increased plasma glucose levels in a Type I diabetes indication of hyperglycemia. It can be seen in Figure 1 that Insulin (1 unit and 3 unit) and P-5-P significantly reduced the plasma glucose levels when compared to diabetic group. Moreover a decreased dose of insulin was needed in the presence of P-5-P to produce the same effect as with a full dose of 3 units of insulin.

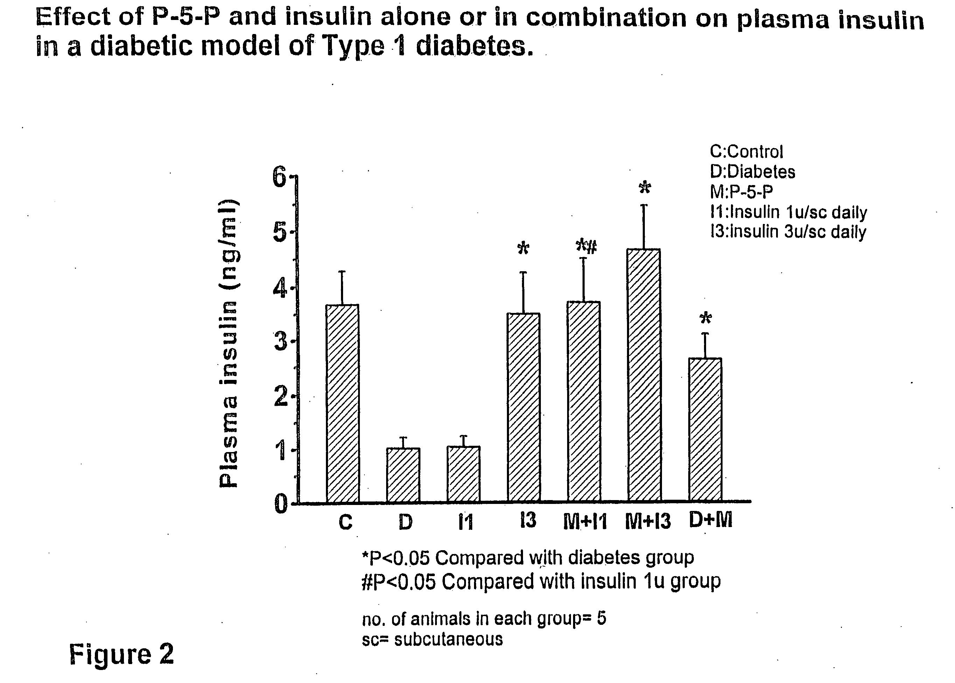

Figure 2 demonstrates the effect of P-5-P and insulin alone or in combination on plasma glucose levels in a Type I diabetes indication of hyperglycemia. It can be seen in Figure 2 that Insulin (1 unit and 3 unit) and P-5-P significantly increased the plasma insulin levels when compared to diabetic group. Moreover a decreased dose of insulin was needed in the presence of P-5-P to produce the same effect as with a full dose of 3 units of insulin.

### Example 10: Control of Glucose and Insulin Levels in Subjects Having Type II Diabetes by Administration of Pyridoxal-5'-Phosphate

To determine the effect of a compound suitable for use in methods of the invention on glucose and insulin levels, as well as increases in systolic blood pressure, rats having type II diabetes are administered pyridoxal-5'-phosphate, either alone or in combination with sucrose and/or tolbutamide, and their systolic blood pressure, urine and blood glucose and insulin levels are determined. Acarbose reduces blood pressure in sucrose induced hypertension in rats. Madar Z et al: Isr J Med Sci; 33:153-159.

As described by Madar et al., Acarbose reduces blood pressure in sucrose induced hypertension in rats. Madar Z et al: Isr J Med Sci; 33:153-159, a high sucrose or fructose diet for a prolonged period is one technique used to induce Type II diabetes, specifically hypertension associated with hyperglycemia and hyperinsulinemia in animals.

Male Sprague-Dawley (Charles River Laboratories, Montreal, Canada) rats weighing approximately 200g are randomly separated into the following groups with each group having 5 amimals:
a) The control group that was fed a normal diet and provided with drinking water
b) The sucrose group that was fed 35% sucrose (35gm sucrose/100 ml of drinking water/day) with an average intake of 150 ml/rat/day
c) The sucrose + P-5-P group that was fed sucrose as stated in b above and 25 mg/kg orally/day of P-5-P
d) The sucrose + tolbutamide group that was fed sucrose as stated in b above and administered 40 mg/kg orally/day of tolbutamide
e) The sucrose + P-5-P + tolbutamide group that was fed sucrose as stated in b above, 25 mg/kg orally/day of P-5-P, and administered 40 mg/kg orally/day of tolbutamide
f) The sucrose + P-5-P + tolbutamide group that was fed sucrose as stated in b above, 25 mg/kg orally/day of P-5-P, and administered 20 mg/kg, orally/day of tolbutamide
g) The sucrose + tolbutamide group that was fed sucrose as stated in b above and 20 mg/kg orally/day of tolbutamide.

Total duration of the study was 16 weeks. Plasma insulin levels were measured using Rat Insulin RIA Kit (Linco Research Inc., St. Charles, MO). Plasma glucose levels were done using the Infinity Glucose Reagent® (Sigma Diagnostics, St. Louis, MO). Blood pressure was measured using the tail cuff method, (see, Madar et al., Acarbose reduces blood pressure in sucrose induced hypertension in rats. Madar Z et al: Isr J Med Sci; 33:153-159).

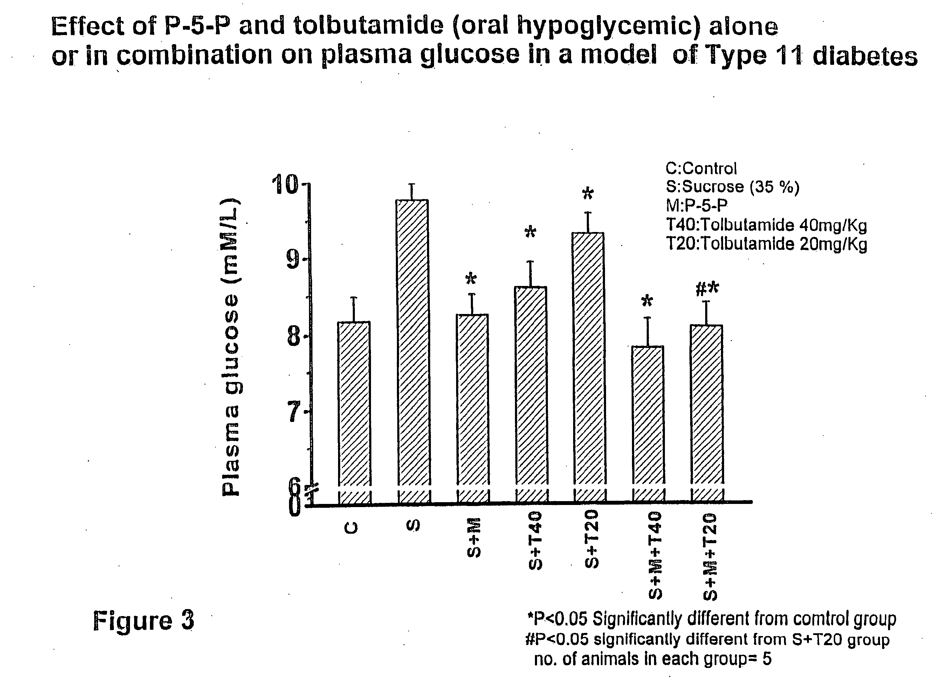

Figure 3 demonstrates the effect of P-5-P and tolbutamide alone or in combination on increased plasma glucose levels in a Type II diabetes indication of hyperglycemia. It can be seen in Figure 3 that tolbutamide (40 and 20 mg/kg) and P-5-P significantly decreased the plasma glucose levels when compared to diabetic group. Moreover a decreased dose of tolbutamide was needed in the presence of P-5-P to produce the same effect as with a full dose of 40 mg/kg tolbutamide.

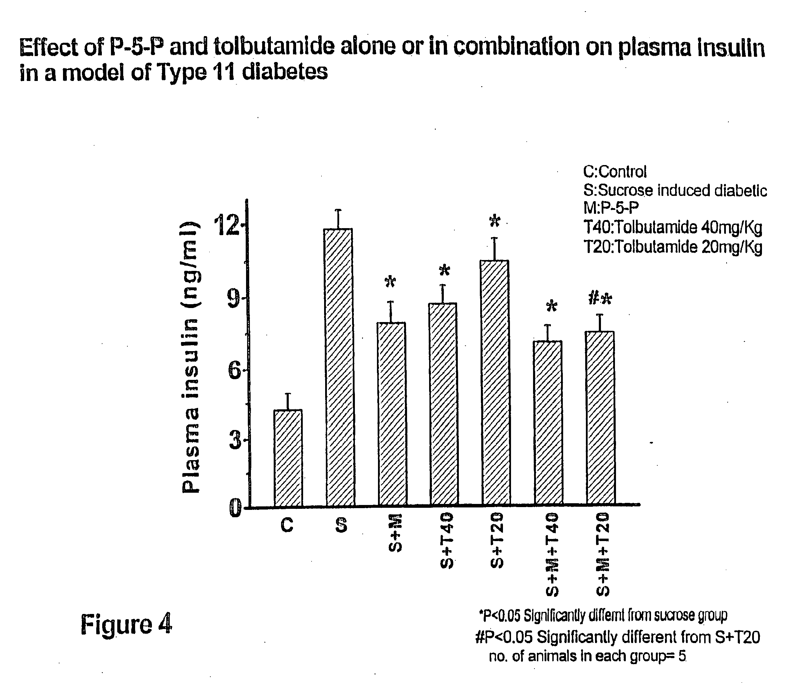

Figure 4 demonstrates the effect of P-5-P and tolbutamide alone or in combination on increased plasma insulin levels in a Type II diabetes indication of hyperglycemia. It can be seen in Figure 4 that tolbutamide (40 and 20 mg/kg) and P-5-P significantly decreased the plasma insulin levels when compared to diabetic group. Moreover a decreased dose of tolbutamide was needed in the presence of P-5-P to produce the same or better effect as with a full dose of 40 mg/kg tolbutamide.

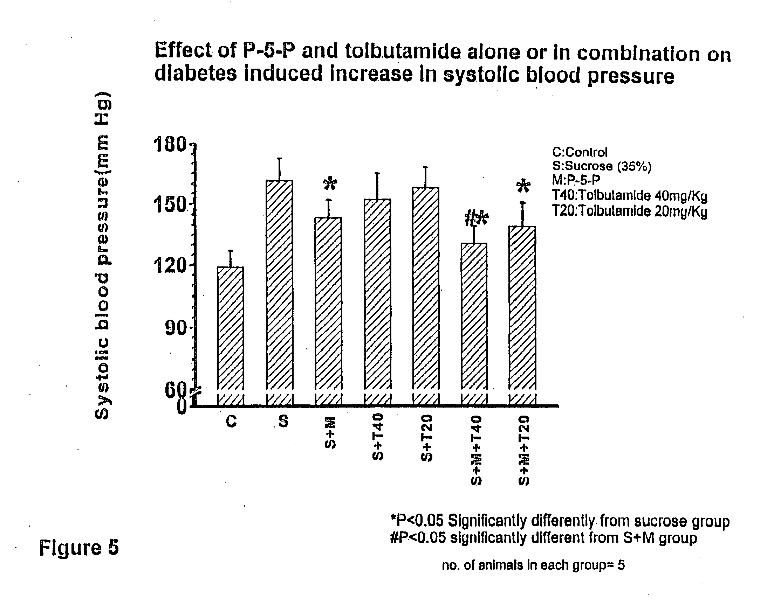

Figure 5 demonstrates the effect of P-5-P and tolbutamide alone or in combination on increased systolic blood pressure in a Type II diabetes indication of hyperglycemia. It can be seen in Figure 5 that that P-5-P significantly decreased the rise in systolic blood pressure when compared to diabetic and tolbutamide treatment groups. Moreover when the rats were treated with P-5-P and tolbutamide (40 mg/kg), the decrease in systolic blood pressure was significantly greater when compared to rats treated with P-5-P or tolbutamide alone.

It should be noted that, as used in the specification and the appended claims, the singular forms "a," "an," and "the' include plural referents unless the content clearly dictates otherwise. Thus, for example, reference to a composition containing "a compound" includes a mixture of two or more compounds. Similarly, reference to "a compound" includes a mixture having more than one compound.

Although embodiments of the invention have been described above, it is not limited thereto, and it will be apparent to persons skilled in the art that numerous modifications and variations form part of the present invention insofar as they do not depart from the spirit, nature, and scope of the claimed and described invention.

## Claims

1. Use of a 3-acylated pyridoxal analogue, a pharmaceutically acceptable acid addition salt thereof, or a mixture thereof for the preparation of a medicament for treating insulin-dependent or non-insulin-dependent diabetes mellitus, related conditions, symptoms of insulin-dependent or non-insulin-dependent diabetes mellitus, or related conditions, or any combination thereof in a mammal.

2. The use of claim 1, wherein the 3-acylated pyridoxal analogue is a compound of the formula 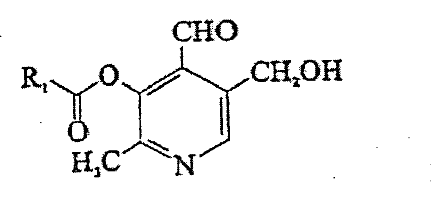 wherein
R₁ is a straight or branched alkyl group of 1 to 8 carbon atoms, a straight or branched alkenyl group of 2 to 8 carbon atoms, in which an alkyl or alkenyl group may be interrupted by a nitrogen or oxygen atom; an alkoxy group of 1 to 4 carbon atoms; a dialkylamino group; an unsubstituted aryl group; or an aryl substituted with an alkyl of 1 to 4 carbon atoms, an alkoxy of 1 to 4 carbon atoms, a hydroxy, an amino, or an acetyloxy group.

3. The use of claim 1, wherein the 3-acylated pyridoxal analogue is a compound of the formula 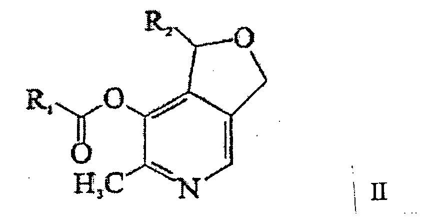 wherein
R₁ is a straight or branched alkyl group of 1 to 8 carbon atoms, a straight or branched alkenyl group of 2 to 8 carbon atoms, in which an alkyl or alkenyl group may be interrupted by a nitrogen or oxygen atom; an alkoxy group of 1 to 4 carbon atoms; a dialkylamino group; an unsubstituted aryl group; or an aryl substituted with an alkyl of 1 to 4 carbon atoms, an, alkoxy of 1 to 4 carbon atoms, a hydroxy, an amino, or an acetyloxy group; and
R₂ is a secondary amino group of the formula 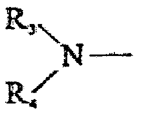 wherein R₃ and R₄ are each independently alkyl of 1 to 8 carbon atoms or when taken together form a ring with the nitrogen atom and which ring may optionally be interrupted by a nitrogen or oxygen atom.

4. The use of claim 2 or 3, wherein R₁ is phenyl or naphthyl in which phenyl or naphthyl is unsubstituted or substituted by an alkyl group of 1 to 4 carbon atoms, an alkoxy group of 1 to 4 carbon atoms, an amino group, a hydroxy group, or an acetyloxy group.

5. The use of claim 2, wherein the 3-acylated pyridoxal analogue is 2-methyl-3-toluoyloxy-4-formyl-5-hydroxymethylpyridine.

6. The use of claim 2, wherein the 3-acylated pyridoxal analogue is 2-methyl-3-beta - naphthoyloxy-4-formyl-5-hydroxymethylpyridine.

7. The use of claim 3, wherein the 3-acylated pyridoxal analogue is 1-morpholino-1,3-dihydro-7-(p-toluoyloxy)-6-methylfuro(3,4-c)pyridine.

8. The use of claim 3, wherein the 3-acylated pyridoxal analogue is 1-morpholino-1,3-dihydro-7-(beta-naphthoyloxy)-6-methylfuro(3,4-c)pyridine.

9. The use of claim 3, wherein the 3-acylated pyridoxal analogue is 1-morpholino-1,3-dihydro-7-pivaloyloxy-6-methylfuro(3,4-c)pyridine.

10. The use of claim 3, wherein the 3-acylated pyridoxal analogue is 1-morpholino-1,3-dihydro-7-(dimethylcarbamoyloxy-6-methylfuro(3,4-c)pyridine.

11. The use of claim 3, wherein the 3-acylated pyridoxal analogue is 1-morpholino-1,3-dihydro-7-acetylsalicyloxy-6-methylfuro(3,4-c)pyridine.

12. The use of any one of claims 1-11, wherein said medicament further comprises a therapeutically effective amount of insulin, a hypoglycemic compound, or a combination thereof.

13. The use of claim 12, wherein the hypoglycemic compound is metformin, acarbose, acetohexamide, glimepiride, tolazamide, glipizide, glyburide, tolbutamide, chlorpropamide, thiazolidinediones, alpha glucosidase inhibitors, biguanindine derivatives, and troglitazone, or a mixture thereof.

14. The use of any one of claims 1-13, wherein insulin-dependent or non-insulin-dependent diabetes mellitus and related conditions and symptoms include diabetes mellitus type I, diabetes mellitus type II, obesity, insulin resistance, hyperinsulinemia, diabetes-induced hypertension, and diabetes-related damage to blood vessels, eyes, kidneys, nerves, autonomic nervous system, skin, connective tissue, or immune system.

15. The use of any one of claims 1-14, wherein the therapeutically effective amount is in a range of about 0.5-100 mg/kg of the mammal's body weight per day.

16. Use of a composition for preparation of a medicament for treating insulin-dependent diabetes mellitus, related conditions, symptoms, or a combination thereof, in a mammal, wherein the composition comprises:
a) 3-acylated pyridoxal analogue, a pharmaceutically acceptable acid addition salt thereof, or mixture thereof, in about 0.5 to about 50 mg/kg of the mammal's body weight per day; and
b) a hypoglycemic compound and optionally
c) insulin.

17. Use of a composition for preparation of a medicament for treating non-insulin-dependent diabetes mellitus, related conditions, symptoms, or a combination thereof, in a mammal, wherein the composition comprises:
a) 3-acylated pyridoxal analogue, a pharmaceutically acceptable acid addition salt thereof, or mixture thereof, in about 0.5 to about 50 mg/kg of the mammal's body weight per day;
b) a hypoglycemic compound; and optionally
c) insulin.
